# FASCICULE DE BREVET EUROPEEN

(11) **EP 4 015 029 B1**
(45) Date de publication et mention de la délivrance du brevet: **02.09.2026**
(21) Numéro de dépôt: 21213059.5
(22) Date de dépôt: 08.12.2021
(51) Int. Cl.: A61M 35/00, A45D 34/00, A45D 34/04

(54) **BOITIER D'APPLICATEUR ET APPLICATEUR DE PRODUIT DERMATOLOGIQUE RECHARGEABLE ET PROCÉDÉ**
APPLIKATORBEHÄLTER UND APPLIKATOR FÜR EIN NACHFÜLLBARES DERMATOLOGISCHES PRODUKT UND VERFAHREN
HOUSING FOR APPLICATOR AND RECHARGEABLE DERMATOLOGICAL PRODUCT APPLICATOR AND METHOD

(30) Priorité: 08.12.2020 FR 2012837
(43) Date de publication de la demande: 22.06.2022
(73) Titulaire: Lightinderm, 75017 Paris (FR)
(72) Inventeur: LAVAL, Sébastien, 75019 PARIS (FR); DECAUX, Géraldine, 75017 PARIS (FR)
(74) Mandataire: Fache, Sébastien

(56) Documents cités:
- WO-A1-2014/091035
- WO-A1-2016/033202
- WO-A1-2017/174918
- KR-A- 20180 099 003
- US-A1- 2016 213 834

## Description

L'invention s'inscrit dans le secteur de la dermatologie et de la cosmétique.

L'invention s'inscrit plus particulièrement dans le secteur de l'instrumentation cutanée et propose un applicateur de produit dermatologique et/ou cosmétique pouvant être chargé avec une cartouche de produit.

De tels applicateurs sont bien connus, en particulier de la publication WO 2014/091035, et sont prévus pour fonctionner avec des recharges - ou capsules - de produit amovibles. Ces recharges comprennent classiquement une tête d'application du produit et un réservoir dont la sortie débouche vers la tête d'application et dont le fond est formé par un piston. L'applicateur comprend en outre des moyens de déplacement du piston pour permettre l'éjection d'une dose prédéterminée de produit au niveau de la tête d'application.

Ces applicateurs rechargeables présentent de nombreux avantages, d'un point de vue économique, hygiénique en cas d'une pluralité d'utilisateurs, et également parce qu'ils permettent l'utilisation d'une variété de produits avec des principes actifs adaptés.

Cependant, un tel applicateur ne détecte pas l'état de remplissage de la recharge insérée. Ainsi, les moyens de déplacement de l'applicateur risquent de buter contre le piston d'une capsule vide, lequel piston est dans ce cas bloqué dans le réservoir de la capsule. Il en résulte un risque de détérioration de l'appareil dans ces conditions d'utilisation.

La publication WO 2017/174918 divulgue un ensemble de distribution de produit fluide qui comprend un réservoir de produit muni d'un piston sur lequel est solidarisé un aimant, lequel piston est déplaçable dans le réservoir à mesure que le fluide est distribué par l'intermédiaire d'une pompe manuelle. Cet ensemble comprend un ou plusieurs capteurs de champ magnétique apte à détecter la position du piston quand ce dernier est en regard dudit capteur.

Cependant, cette solution ne permet de connaitre que quelques positions prédéterminées du piston dans le réservoir.

La publication WO 2016/033202 divulgue un applicateur de produit consommable muni d'une ou plusieurs cartouches comprenant le produit à distribuer. Chaque cartouche comprend un piston déplaçable dans un réservoir de produit, et le réservoir est relié à une pompe électrique déportée qui crée une surpression dans le réservoir assurant le déplacement du piston et la distribution du produit. En outre, l'applicateur est muni d'un capteur optique de détection de la surface arrière du piston.

Cependant, un tel agencement pneumatique ne permet pas une distribution précise de produit, et le capteur optique est uniquement adapté à la configuration de l'applicateur décrit dans cette publication.

L'invention est définie par les revendications 1 et 11 et définie plus avant par les revendications dépendantes 2-10 et 12-14. L'invention a ainsi pour objet de proposer un applicateur rechargeable de produit dermatologique permettant de détecter l'état de remplissage de la capsule de produit insérée dans l'applicateur y compris lorsque les moyens de déplacement du piston sont au contact dudit piston. Le dispositif doit permettre une détection précise quel que soit la position du piston, doit pouvoir être réalisé avec des composants à coûts raisonnable et doit pouvoir être installé en des endroits protégés de l'appareil tout en tenant compte de la présence des moyens motorisé de déplacement du piston.

À cet effet, l'invention vise un boitier d'applicateur de produit dermatologique rechargeable s'étendant selon un axe longitudinal, apte à intégrer de manière amovible une capsule comprenant une tête d'application de produit située au niveau d'une extrémité libre du boitier, et un réservoir de produit dermatologique relié à la tête d'application et comprenant, au niveau d'une de ses extrémités, une sortie de produit débouchant en vis-à-vis de la tête d'application et, au niveau de son extrémité opposée, un piston formant le fond du réservoir, lequel piston est déplaçable en translation dans le réservoir selon l'axe longitudinal pour assurer la sortie de produit hors du réservoir vers la tête d'application, lequel boitier comprend un corps principal s'étendant selon l'axe longitudinal et des moyens de déplacement motorisés du piston ménagés dans le corps principal et comprenant un élément pousse-piston déplaçable en translation selon l'axe longitudinal et dont l'extrémité libre est prévue pour venir en appui de contact contre le piston du réservoir, lesquels moyens de déplacement comprennent des moyens de détection du piston de la capsule et de détermination de sa position dans le réservoir au moins lorsque l'élément pousse-piston est au contact du piston, lequel boitier d'applicateur comprend des moyens de contrôle et de commande desdits moyens de déplacement configurés pour corréler la position du piston avec la quantité de produit dermatologique restant dans le réservoir.

Le boitier d'applicateur peut également comporter les caractéristiques optionnelles suivantes considérées isolément ou selon toutes les combinaisons techniques possibles :
- Les moyens de détection de la position du piston sont configurés pour détecter une surconsommation du moteur lorsque l'élément pousse-piston rentre en appui de contact contre le piston.
- Les moyens de détection du piston comprennent un détecteur de fin de course solidaire de l'extrémité libre de l'élément pousse-piston et configuré pour détecter un contact avec le piston.
- Les moyens de détermination de position du piston sont configurés pour déterminer la longueur de déplacement de l'élément pousse-piston entre une position initiale et une position dans laquelle l'élément pousse-piston est configurée pour être au contact du piston.
- Les moyens de détermination de position comprennent un capteur du type codeur optique ménagé sur les moyens de déplacement et configuré pour mesurer la distance parcourue par ledit élément pousse-piston.
- Les moyens de détermination de la position comprennent un aimant et un capteur du type à effet Hall de lecture de l'aimant solidaire de l'élément pousse-piston.
- L'élément pousse-piston est monté flottant sur un ressort.
- L'élément pousse-piston est monté autour d'au moins une tige de guidage.
- Les moyens de déplacement comprennent un élément pousseur relié à l'élément pousse-piston et un moteur comportant un axe de rotation fileté, lequel élément pousseur comprend une portion taraudée montée sur l'axe fileté et une fourche radiale anti-rotation coopérant avec un élément anti-rotation solidaire du boitier d'applicateur.

L'invention vise également un applicateur comprenant un boitier d'applicateur tel que décrit précédemment et une capsule intégrée de manière amovible dans le corps principal et comprenant une tête d'application de produit située au niveau de l'extrémité libre du corps principal, et un réservoir de produit dermatologique relié à la tête d'application et comprenant, au niveau de l'une de ses extrémités, une sortie de produit débouchant en vis-à-vis de la tête d'application et, au niveau de son extrémité opposée, un piston formant le fond du réservoir, lequel piston est déplaçable en translation dans le réservoir selon l'axe longitudinal pour assurer la sortie de produit dermatologique hors dudit réservoir vers la tête d'application.

L'invention vise également un procédé de contrôle de la quantité d'un produit dermatologique contenu dans un réservoir muni d'un piston d'une capsule pour un applicateur rechargeable de produit dermatologique, lequel applicateur comprend un corps principal s'étendant selon un axe longitudinal et des moyens de déplacement motorisés du piston du réservoir ménagés dans le corps principal et comprenant un élément pousse-piston déplaçable en translation selon l'axe longitudinal et dont l'extrémité libre est prévue pour venir en appui de contact contre le piston du réservoir, lequel procédé comprend les étapes successives de :
- Insertion d'une capsule de produit dermatologique dans le corps principal de l'applicateur, la capsule comportant une tête d'application de produit ménagée au niveau d'une extrémité libre du corps principal et le réservoir de produit relié à la tête d'application et comprenant, au niveau d'une de ses extrémités, une sortie de produit et, au niveau de son extrémité opposée, le piston formant le fond du réservoir, lequel piston est déplaçable en translation dans le réservoir selon l'axe longitudinal pour assurer la sortie de produit hors dudit réservoir ;
- Actionnement par des moyens de commande de l'applicateur, des moyens de déplacement en direction du piston ;
- Détection du contact entre les moyens de déplacement et le piston, suivi de l'arrêt des moyens de déplacement ;
- Détermination de la position du piston et de la quantité de produit cosmétique dans le réservoir à partir de ladite position du piston.

Le procédé peut également comporter les caractéristiques optionnelles suivantes considérées isolément ou selon toutes les combinaisons techniques possibles :
- Le procédé comprend une étape de déplacement des moyens de déplacement vers une position initiale dès que le réservoir est dégagé du corps principal.
- L'étape de détermination de la position du piston comprend les sous-étapes de :
   - Détermination de la distance parcourue par les moyens de déplacement entre leur position initiale et leur position de contact avec le piston ;
   - Corrélation par les moyens de contrôle et de commande entre la distance parcourue par les moyens de déplacement et une base de données enregistrée dans un espace mémoire des moyens de contrôle et de commande pour déterminer la position du piston et la quantité de produit restant dans le réservoir.
- L'applicateur comprend des moyens de détection du piston et de détermination de sa position dans le réservoir, et l'actionnement des moyens de déplacement en direction du piston comprend les sous-étapes de :
   - Détermination préliminaire de la position du piston dans le réservoir
   - Actionnement et déplacement des moyens de déplacement selon une première vitesse déterminée ;
   - Détection des moyens de déplacement à une distance du piston inférieure à une valeur déterminée, et,
   - Diminution de la vitesse de progression des moyens de déplacement jusqu'à une seconde vitesse déterminée.

D'autres caractéristiques et avantages de l'invention ressortiront clairement de la description qui en est donnée ci-dessous, à titre indicatif et nullement limitatif, en référence aux figures annexées :
[Fig 1] la figure 1 représente une vue en coupe longitudinale d'une partie de l'applicateur selon un premier mode de réalisation ;
[Fig 2] la figure 2 représente une vue en coupe longitudinale d'une partie de l'applicateur selon un second mode de réalisation ;
[Fig 3] la figure 3 représente une autre vue en coupe longitudinale de l'applicateur de la figure 2.

Il est tout d'abord précisé que sur les figures, les mêmes références désignent les mêmes éléments quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

Il est également précisé que les figures représentent essentiellement deux modes de réalisation de l'objet de l'invention mais qu'il peut exister d'autres modes de réalisation qui répondent à la définition de l'invention.

L'invention concerne un applicateur 1, 1a de produit dermatologique rechargeable, c'est-à-dire comprenant un boitier d'applicateur 100, 100a fonctionnant avec des capsules 16, 16a de produit dermatologique interchangeables, et en particulier un applicateur de produit cosmétique rechargeable. Le boitier d'applicateur 100, 100a et la capsule 16, 16a intégrée dans ledit boitier 100, 100a forment ensemble l'applicateur 1, 1a. Il est bien entendu que la capsule 16, 16a peut comprendre n'importe quel produit cosmétique ou dermatologique prévu pour une application cutanée.

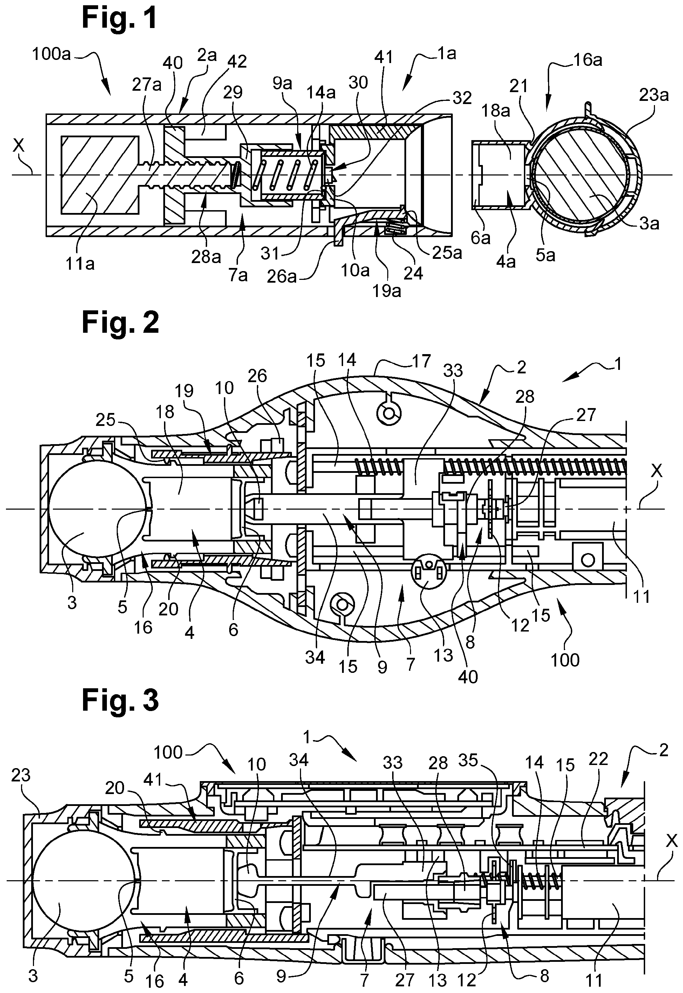

En référence aux figures 1 à 3, qui représentent une partie avant de l'applicateur 1, 1a, c'est-à-dire une partie comprenant des moyens pour appliquer un produit cosmétique sur la peau d'un utilisateur, ledit applicateur 1, 1a comprend un corps principal 2, 2a de forme générale cylindrique qui s'étend selon un axe longitudinal X. Avantageusement et comme représenté sur la figure 3, le corps principal comprend une zone de préhension 17 plus large, pour faciliter la prise en main.

L'applicateur 1, 1a comprend en outre une capsule de produit 16, 16a intégrée de manière amovible dans le corps principal 2, 2a. Cette capsule 16, 16a comprend un réservoir de produit 4, 4a et une bille 3, 3a montée à rotation au niveau d'une des extrémités du réservoir 4, 4a pour former une tête d'application de produit sur la peau de l'utilisateur. Le réservoir 4, 4a comprend en outre une cavité cylindrique 18, 18a qui comprend une sortie de produit 5, 5a débouchant en vis-à-vis de la bille 3, 3a, et une paroi de fond 6, 6a formée par un piston mobile en translation et disposé au niveau de l'extrémité opposée du réservoir 4, 4a.

La capsule 16, 16a est insérable dans le corps principal 2, 2a de l'applicateur 1, 1a dans un orifice ménagé au niveau de l'extrémité avant dudit corps 2, 2a, et plus particulièrement dans un siège d'accueil 41 du réservoir 18 de capsule. Cette capsule est maintenue solidaire du corps principal 2, 2a par engagement élastique via un ou plusieurs éléments de liaison 19, 19a (figure 2) montés dans l'épaisseur de paroi cylindrique du siège d'accueil 41 (figure 3) du corps principal 2, 2a et dont les extrémités coopèrent avec une collerette 20 (figures 2 et 3), des saillies ou encore des encoches 21 (figure 1) ménagées sur la paroi du réservoir 4, 4a de la capsule 16, 16a. Une fois insérée dans le corps principal 2, 2a, la capsule 16, 16a est alignée selon l'axe longitudinal X et le piston 6, 6a du réservoir 4, 4a est mobile en translation selon cet axe longitudinal X.

L'applicateur 1, 1a comprend également des moyens de déplacement motorisés 7, 7a du piston 6, 6a de la capsule 16, 16a, qui seront décrits précisément ci-dessous en lien avec les différents modes de réalisation de l'invention. En d'autres termes, le boitier d'applicateur 100, 100a comprend un moteur 11, 11a relié aux moyens de déplacement 7, 7a pour les motoriser. L'applicateur 1, 1a comprend également des moyens de contrôle et de commande, en particulier une carte électronique 22 comportant au moins une unité de traitement et un espace mémoire, pilotant les moyens de déplacement motorisés 7, 7a. Dans la suite de la description, l'expression « carte électronique » sera utilisée pour définir les moyens de contrôle et de commande.

L'applicateur 1, 1a comprend en outre un dispositif de commande manuelle (non représenté) ménagé sur le corps principal 2, 2a, le cas échéant au niveau des moyens de préhension 17, et relié électriquement à la carte électronique 22. L'utilisateur peut ainsi contrôler la mise en veille et la mise sous tension de l'applicateur, ainsi que la distribution de produit cosmétique sur la peau : à chaque commande de distribution de produit engagée par l'utilisateur via le dispositif de commande manuelle, la carte électronique 22 pilote les moyens de déplacement motorisés 7, 7a pour distribuer une dose déterminée de produit cosmétique.

L'applicateur peut également comprendre des moyens d'illumination (non représentés), par exemple une diode électroluminescente reliée à la carte électronique 22 et dont la mise en veille et la mise sous tension sont commandées manuellement par l'utilisateur via le dispositif de commande dédié. Cette diode assure l'émission de lumière au travers et/ou en périphérie de la bille 3, 3a en direction de la peau de l'utilisateur. Dans ces conditions, la bille 3, 3a est donc transparente aux longueurs d'ondes d'émission de la diode.

Enfin, l'applicateur 1, 1a peut le cas échéant comprendre un capot amovible 23, 23a coopérant avec au moins un élément de fixation par engagement élastique ménagé au niveau de l'extrémité libre avant du corps principal (figures 2 et 3) ou au niveau de l'extrémité avant du réservoir de la capsule (figure 1), lequel capot 23, 23a est destiné à protéger la bille d'application 3, 3a quand l'applicateur 1, 1a n'est pas utilisé.

En référence à la figure 1, l'applicateur 1a va être décrit selon un premier mode de réalisation.

Dans ce mode de réalisation, la capsule 16a est amoviblement fixée au corps principal 2a par un élément 19a monté sur ressort 24 et dont l'extrémité libre 25a en forme de crochet coopère avec une encoche 21 ménagée dans la capsule 16a. Lorsque la capsule 16a est insérée dans le corps principal 2a, la tension du ressort 24 maintient le crochet 25a engagé dans l'encoche 21. Pour désengager la capsule 16a, l'utilisateur exerce une pression sur l'extrémité opposée 26a de l'élément 19a qui fait saillie du corps principal 2a, laquelle pression provoque un mouvement de la partie d'extrémité libre opposée qui comprime le ressort 24 et désengage le crochet 25a de l'encoche considérée 21. Une fois que l'utilisateur relâche la pression sur l'extrémité libre 26a de l'élément 19a, le ressort 24 reprend sa position initiale.

L'applicateur 1a comprend un moteur 11a relié électriquement à la carte électronique (non représentée), lequel moteur 11a comprend un axe rotatif fileté 27a. L'applicateur 1a comprend également un élément pousseur 28a comportant une première partie creuse taraudée montée sur l'axe fileté, et une fourche radiale 40 anti-rotation coopérant avec un élément anti-rotation 42 solidaire du boitier d'applicateur 100a. Plus précisément, cet élément anti-rotation est une nervure longitudinale 42 ménagée dans le boitier 100a et dans laquelle la fourche radiale 40 est engagée. L'élément pousseur 28a comporte en outre une seconde partie en forme de cylindre creux avec une paroi de fond 29. L'élément pousseur 28a, simplement nommé « pousseur » dans la suite de la description, est donc déplaçable en translation selon l'axe longitudinal X lorsque l'axe moteur 27a est mis en rotation. Selon le sens de rotation du moteur 11, le pousseur 28a soit se rapproche de la capsule 16a, soit s'éloigne de cette dernière.

Le pousseur 28a comprend également un ressort 14a sur lequel est monté flottant un élément pousse-piston 9a. Cet élément pousse-piston 9a, simplement nommé « pousse-piston » dans la suite de la description, présente la forme d'un cylindre creux avec une paroi de fond formant son extrémité libre 10a : le ressort est donc introduit dans le pousse-piston 9a, et ses extrémités respectives sont solidaires de la paroi de fond 29 du pousseur 28a et de la face interne de la paroi de fond 10a du pousse-piston 9a. Ce ressort 14a exerce une force longitudinale sur le pousse-piston 9a en direction du piston 6a de la capsule 16a, sans que la force exercée par ledit ressort 14a ne soit suffisante pour provoquer le déplacement dudit piston 6a dans le réservoir 4a.

Le pousseur 28a, le ressort 14a et le pousse-piston 9a sont tous coaxiaux selon l'axe longitudinal X.

Enfin, l'applicateur 1a comprend un détecteur de piston 30 comprenant un circuit électrique 31 monté contre la face interne de la paroi de fond 10a du pousse-piston 9a et relié à la carte électronique, et une tête de détection 32 traversant la paroi de fond au travers d'un orifice prévu à cet effet de manière à être en vis-à-vis du piston 6a de la capsule 16a.

Ce détecteur 30 est du type détecteur de fin de course, par exemple un interrupteur à bras de levier ou un détecteur de type dôme, dont la fonction est d'arrêter le moteur 11a dès qu'il détecte un appui de contact contre le piston 6a de la capsule 16a. La force d'activation du détecteur 30 est inférieure à la force nécessaire au déplacement du piston 6a dans le réservoir 4a.

Un procédé de contrôle de la quantité de produit cosmétique présent dans une capsule 16a va maintenant être décrit, en lien avec le premier mode de réalisation de l'invention.

La présence ou non d'une capsule 16 dans le siège d'accueil 41 est repéré par le basculement ou non d'un interrupteur électrique non illustré agencé en périphérie du fond circulaire de siège 41. Si la carte électronique ne détecte aucune capsule insérée 16a, alors elle commande le déplacement du pousseur 28a vers une position dite à vide en retrait, la plus éloignée possible de l'extrémité du corps principal 2a. Les coordonnées de cette position sont connues et enregistrées dans l'espace mémoire de la carte électronique.

Lorsque l'utilisateur met l'applicateur 1a sous tension, la carte électronique vérifie que le détecteur de piston 30 n'envoie aucun signal de détection dudit piston 6a, puis commande l'actionnement du moteur 11a, assurant la rotation de l'axe fileté 27a et le déplacement du pousseur 28a en direction du piston 6a. Concomitamment au déclenchement de la rotation de l'axe fileté 27a, la carte électronique lance une minuterie.

Dès que l'extrémité libre 10a du pousse-piston 9a vient au contact du piston 6a, au point que la force exercée l'un sur l'autre atteint la valeur seuil de détection du détecteur considéré 30, ce dernier envoie un signal à la carte électronique qui commande en retour l'arrêt du moteur 11a et l'arrêt de la minuterie. Le temps de fonctionnement du moteur 11a est alors déterminé par la carte électronique.

La vitesse de progression du pousseur 28a étant connue et enregistrée dans une base de données enregistrée en mémoire de la carte électronique (vitesse déterminée à partir de la vitesse de rotation de l'axe filetée 27a et du pas de filetage), la carte électronique détermine la distance parcourue par le pousseur 28a jusqu'à la détection du piston 6a, et détermine de fait une position de l'extrémité libre 10a du pousse-piston 9a (la taille dudit pousse-piston 9a étant connue) et donc celle du piston 6a de la capsule 16a, quelle que soit la position dudit piston 6a dans le réservoir 4a.

La carte électronique comprenant dans sa base de données des tables corrélant la position du piston 6a avec le nombre de doses de produit cosmétique restant dans la capsule 16a, la carte électronique détermine alors le nombre de doses restantes dans le réservoir 4a à partir de la position du piston 6a déterminée.

L'utilisateur peut alors déclencher un cycle de traitement, le moteur tournant à une vitesse permettant l'avancée du pousseur, donc du pousse piston donc du piston de capsule délivrant une dose en un temps prédéterminée, par exemple en 30 secondes. Suite à ce traitement, l'utilisateur peut sortir sa capsule de l'applicateur pour laisser celui-ci à disposition d'un membre de la famille utilisant une autre capsule. Lorsqu'il voudra reprendre son traitement un jour suivant, l'utilisateur insérera sa propre capsule dans l'applicateur qui commencera une nouvelle détection de position actuelle du piston dans ladite capsule.

Ainsi, l'applicateur 1a selon l'invention permet de déterminer en temps réel la position du piston 6a dans le réservoir 4a et la quantité de produit restant dans la capsule 16a sans outils ou capteurs de mesure supplémentaire, puisque le seul détecteur 30 couplé à la carte électronique suffisent à obtenir ces données.

Dans ce mode de réalisation, on pourrait imaginer que le pousseur 28a et le pousse piston 9a soient monobloc sans le ressort 14a. Toutefois, les moyens électroniques de pilotage du moteur 11a doivent alors être particulièrement réactif au signal de fermeture du détecteur 30.

En référence aux figures 2 et 3, l'applicateur 1 va être décrit selon un second mode de réalisation.

Dans ce mode de réalisation, la capsule 16 est amoviblement fixée au corps principal 2 par deux éléments 19 montés sur ressorts (non représentés) dans l'épaisseur de paroi cylindrique du siège d'accueil 41 et dont les extrémités libres en forme de crochets 25 coopèrent avec une collerette 20 ménagée autour du réservoir 4. La tension du ressort maintient le crochet 25 engagé sur la collerette 20. Pour désengager la capsule 16, l'utilisateur exerce une pression sur les extrémités opposées 26 des éléments 19 en exerçant une pression sur les moyens de préhension 17 du corps principal 2, laquelle pression provoque un mouvement de l'élément considéré 19 qui désengage le crochet 25 de la collerette 20. Une fois que l'utilisateur relâche la pression sur les extrémités libres 26 des éléments 19, les ressorts reprennent leur position initiale.

L'applicateur 1 comprend un moteur 11 relié électriquement à la carte électronique 22, lequel moteur 11 comprend un axe rotatif fileté 27. L'applicateur 1 comprend également un élément pousseur 28 - simplement nommé « pousseur » dans la suite de la description - comportant un orifice taraudé traversant monté sur l'axe fileté 27 et une fourche radiale anti-rotation 40 coopérant avec un élément anti-rotation 15 solidaire du boitier d'applicateur 100. Plus précisément, cet élément anti-rotation est une tige de guidage 15 solidaire du corps principal 2 du boitier 100 et dans laquelle est engagée la fourche radiale 40. Le pousseur 28 est donc déplaçable en translation selon l'axe longitudinal X lorsque l'axe moteur 27 est mis en rotation. Selon le sens de rotation du moteur 11, le pousseur 28 soit se rapproche de la capsule 16, soit s'en éloigne.

Le pousseur 28 est en outre disposé en vis-à-vis d'un élément pousse-piston 9 - appelé simplement « pousse-piston » dans la suite de la description - comprenant une première partie 33 montée sur deux tiges de guidage parallèles 15 intégrées dans le corps principal 2 et s'étendant selon l'axe longitudinal X, et une seconde partie 34 dont l'extrémité libre 10 est prévue pour venir au contact du piston 6 de la capsule 16. Ainsi, le mouvement en translation du pousseur 28 induit par la rotation de l'axe fileté 27 provoque le déplacement du pousse-piston 9 dans la même direction.

Au moins une des tiges de guidage 15 comprend un ressort 14 monté coaxialement autour de la tige considérée 15, lequel ressort 14 exerce une force longitudinale sur le pousse-piston 9 en direction du piston 6 de la capsule 16, sans que la force exercée par ledit ressort 14 ne soit suffisante pour provoquer le déplacement dudit piston 6 dans le réservoir 4.

L'applicateur 1 comprend un élément de détection préalable de la position du pousse-piston 9 dans le corps principal 2. Cet élément de détection préalable comprend un aimant 13 solidaire du pousse-piston 9 et un détecteur magnétique (non représenté) relié à la carte électronique 22, par exemple un capteur à effet Hall, mesurant la variation de champs magnétique induite par le mouvement de l'aimant 13 avec une précision de déplacement du pousse-piston 9 de l'ordre de 1 mm.

Un tel élément de détection magnétique peut en outre être intégré dans l'applicateur 1a selon le premier mode de réalisation.

L'applicateur 1 selon le second mode de réalisation de l'invention comprend enfin un détecteur de position fine 8 du pousse-piston 9. Ce détecteur 8 est du type détecteur optique et comprend au moins une roue à codage optique 12 solidaire de l'axe fileté 27 et une fourche de lecture optique 35 reliée à la carte électronique 22 et configurée pour lire les informations de la roue de codage 12. Grâce à des tables contenues dans la base de données enregistrée en mémoire de la carte électronique 22, cette dernière corrèle la lecture de la roue de codage optique 12 avec une position du pousseur 28 et du pousse-piston 9. Les dimensions du pousseur 28 et du pousse-piston 9 étant connues, la carte électronique en déduit la position du piston 6 de la capsule 16, quelle que soit la position dudit piston 6 dans le réservoir 4.

Un procédé de contrôle de la quantité de produit cosmétique présent dans une capsule 16 va maintenant être décrit, en lien avec le second mode de réalisation de l'invention.

Si la carte électronique 22 ne détecte aucune capsule insérée 16, par exemple par le non basculement d'un interrupteur électrique non illustré agencé en périphérie du fond circulaire de siège 41, alors elle commande le déplacement du pousseur 28 vers la position à vide, en retrait, la plus éloignée possible de l'extrémité du corps principal 2. Les coordonnées de cette position sont connues et enregistrées dans l'espace mémoire de la carte électronique 22. De manière avantageuse, le retour vers cette position à vide est garanti par un second détecteur optique (non représenté) à roue de codage et fourche optique, la seconde fourche étant solidaire de la carte électronique 22 et la seconde roue de codage étant solidaire de l'axe fileté 27. En effet, dès que la seconde fourche lit sur la seconde roue de codage l'information correspondant à la position à vide du pousseur 28, la carte électronique 22 stoppe le moteur 11.

Lors de l'insertion de la capsule, le piston 6 de capsule 16 vient en contact avec l'extrémité libre 10 du pousse piston 9 et le fait reculer d'une distance plus ou moins grande selon la position résiduelle de ce piston 6 dans sa capsule. L'aimant 13 solidaire du pousse-piston 9 passe devant le détecteur magnétique (non représenté) relié à la carte électronique 22, (par exemple un capteur à effet Hall) ce qui donne une indication préliminaire de la position du piston 6 dans sa capsule16.

Lorsque l'utilisateur met l'applicateur 1 sous tension, la carte électronique 22 commande l'actionnement du moteur 1, assurant la rotation de l'axe fileté 27 et le déplacement du pousseur 28 en direction du piston 6. Concomitamment au déclenchement de la rotation de l'axe fileté 27, la carte électronique 22 mesure en continue le courant électrique aux bornes du moteur 11.

Dès que la carte électronique 22 détecte une augmentation de la consommation de courant par le moteur électrique 11 supérieure à un seuil déterminé, par exemple une surconsommation de 15% de courant électrique, alors la carte électronique 22 met hors tension le moteur électrique 11 pour stopper la rotation de l'axe filetée 27 et l'avancée du pousse-piston 9, car le pousseur 28 est alors en appui de contact contre le pousse-piston 9. Cette solution offre une grande précision de mise en contact du pousseur 28 contre le pousse-piston 9, de l'ordre de 25 microns. Autrement dit, la distance parcourue par le pousseur 28 et le pousse-piston 9, entre l'instant du contact réel du pousseur 28 contre le pousse-piston 9 et la détection par la carte électronique 22 de ce contact, ne dépasse pas 25 microns ; cela évite donc tout mouvement intempestif du piston 6 de la capsule 16 et donc toute distribution non désirée de produit.

La fourche optique 35 lit alors l'information correspondante sur la roue de codage considérée 12. Des tables contenues dans la base de données enregistrée en mémoire de la carte électronique 22 corrèlent les informations de la roue de codage 12 avec les positions du pousseur 28, du pousse-piston 9 et du piston 6 de la capsule 16. Enfin, la carte électronique 22 comprenant dans sa base de données des tables corrélant la position du piston 6 avec le nombre de doses de produit cosmétique restant dans la capsule 16, la carte électronique 22 détermine alors le nombre de doses restantes dans le réservoir 4 à partir de la position déterminée du piston 6.

En particulier et de manière avantageuse, une valeur lue sur la roue de codage 12 est liée à une position initiale du pousseur 9, elle-même corrélée à une position initiale de piston 6 lorsque le réservoir 4 est plein. Ainsi, le volume total du réservoir 4 et le nombre de doses contenu dans ledit réservoir 4 étant connu, il suffit à la carte électronique 22 d'incrémenter un compteur à chaque dose distribuée pour une capsule 16 pour déterminer le nombre de doses restantes et empêcher la mise en marche du moteur 11 lorsque la carte électronique 22 détecte que la capsule 16 est vide.

En outre, les tables relient le nombre de doses restantes à une position de piston 6, et donc à une valeur particulière lue sur la roue de codage considérée 12. Ainsi, la carte électronique 22 est en mesure de déterminer si la capsule 16 intégrée est pleine ou déjà en partie ou totalement utilisée.

Ainsi, l'applicateur 1 selon l'invention permet de déterminer en temps réel la position du piston 6 dans le réservoir 4 et la quantité de produit restant dans la capsule 16 y compris lorsque la surface du piston 6 n'est pas accessible à une détection et/ou à une mesure directe.

De manière avantageuse et suite à l'insertion d'une capsule 16 dans l'appareil 1, la lecture préliminaire de position permet à la carte électronique 22 de définir de manière approximative la position du piston 6. De fait, la position approximative du piston 6 dans le réservoir est déterminée de manière préliminaire avant l'actionnement des moyens de déplacement 7 en direction du piston 6.

Une fois cette détermination préliminaire de la position du piston 6 réalisée, la carte électronique 22 peut déclencher le moteur 11 d'abord à une première vitesse de rotation relativement élevée pour limiter le temps de rapprochement du pousseur 28 du pousse-piston 9. Les moyens de déplacement 7 se déplacent alors à une première vitesse déterminée.

Puis lorsque la carte électronique détecte que les moyens de déplacement 7 sont à une distance du piston 6 inférieure à une valeur déterminée, et plus précisément que le pousseur (par lecture de la roue de codage 12) est à proximité du pousse-piston 9 (par exemple, d'une distance inférieure ou égale à 3mm), alors ladite carte électronique 22 adapte la vitesse du moteur 11, avec une rotation plus lente de l'axe fileté 27 pour éviter un appui de contact trop brutal du pousse-piston 9 contre le piston 6 de la capsule 16. Les moyens de déplacement 7 se déplacent alors à une seconde vitesse déterminée inférieure à la première vitesse déterminée.

Selon un troisième mode de réalisation non représenté, l'applicateur comprend un boitier d'applicateur présentant une forme de pistolet avec une première partie formant poignée s'étendant selon un premier axe et une seconde partie formant distributeur et s'étendant selon un second axe perpendiculairement à la poignée.

Le distributeur comprend au niveau de son extrémité libre un orifice de réception d'une capsule de produit telle que décrite précédemment, c'est-à-dire comprenant une tête de distribution et un réservoir de produit muni d'un piston déplaçable à translation dans ledit réservoir.

Les moyens de déplacement motorisés du piston comprennent une première crémaillère s'étendant dans la poignée selon le premier axe, et un moteur relié à la crémaillère et assurant son déplacement parallèlement à l'axe de la poignée. En outre, les moyens de déplacement comprennent une seconde crémaillère s'étendant dans le distributeur selon le second axe et formant l'élément pousse-piston. De fait, l'extrémité libre de la seconde crémaillère est prévue pour venir en appui de contact contre le piston de la capsule logée dans le distributeur.

Enfin, les moyens de déplacement comprennent un pignon faisant la liaison mécanique entre les deux crémaillères : le déplacement en translation de la première crémaillère provoque la rotation du pignon, laquelle rotation dudit pignon provoque la translation de la seconde crémaillère dans le distributeur selon le second axe. Le déplacement en translation de la seconde crémaillère et en particulier de son extrémité libre en direction et au contact du piston, provoque le déplacement de ce dernier dans le réservoir de la capsule.

L'applicateur, dans ce troisième mode de réalisation, comprend également des moyens de détection du piston et de détermination de sa position dans le réservoir. Avantageusement, ces moyens de détection et de détermination de la position du piston peuvent comprendre un détecteur de fin de course tel que celui décrit en lien avec le premier mode de réalisation, et/ou un capteur du type codeur optique ménagé au niveau de la première crémaillère ou de la seconde crémaillère, et/ou un aimant et un capteur à effet Hall de lecture de l'aimant solidaire de la première ou seconde crémaillère, tels que décrits en lien avec le second mode de réalisation.

L'applicateur 1, 1a de l'invention peut ainsi être partagé entre plusieurs utilisateurs, chacun pouvant utiliser sa propre capsule 16, 16a quelle que soit la quantité de produit cosmétique restant dans cette capsule 16, 16a. En outre, un même utilisateur peut utiliser des capsules 16, 16a différentes (c'est-à-dire comprenant des produits cosmétiques avec des principes actifs différents) sans attendre qu'une capsule 16, 16a soit totalement vide avant interversion des capsules. Enfin, l'applicateur 1, 1a comprenant une capsule 16, 16a vide de produit est mis en sécurité par la carte électronique 22, et en particulier empêche la mise sous tension du moteur 11, 11a et des moyens d'illumination lorsque l'applicateur 1, 1a comprend de telles moyens d'illumination.

## Revendications

1. Boitier d'applicateur (100, 100a) de produit dermatologique rechargeable s'étendant selon un axe longitudinal (X), apte à intégrer de manière amovible une capsule (16, 16a) comprenant une tête d'application de produit (3, 3a) située au niveau d'une extrémité libre du boitier (100, 100a), et un réservoir de produit dermatologique (4, 4a) relié à la tête d'application (3, 3a) et comprenant, au niveau d'une de ses extrémités, une sortie (5, 5a) de produit débouchant en vis-à-vis de la tête d'application (3, 3a) et, au niveau de son extrémité opposée, un piston (6, 6a) formant le fond du réservoir (4, 4a), lequel piston (6, 6a) est déplaçable en translation dans le réservoir (4, 4a) selon l'axe longitudinal (X) pour assurer la sortie de produit hors du réservoir (4, 4a) vers la tête d'application (3, 3a), lequel boitier (100, 100a) comprend :
• un corps principal (2, 2a) s'étendant selon l'axe longitudinal (X), et
• des moyens de déplacement (7, 7a) motorisés du piston (6, 6a) ménagés dans le corps principal (2, 2a) et comprenant un élément pousse-piston (9, 9a) déplaçable en translation selon l'axe longitudinal (X) et dont l'extrémité libre (10, 10a) est prévue pour venir en appui de contact contre le piston (6, 6a) du réservoir (4, 4a),
**caractérisé en ce que**
les moyens de déplacement (7, 7a) comprennent des moyens de détection du piston (6, 6a) de la capsule (16,16a) et de détermination (8) de sa position dans le réservoir (4, 4a) lorsque l'élément pousse-piston (9, 9a) est au contact du piston (6, 6a), quelle que soit la position dudit piston (6, 6a) dans le réservoir (4, 4a),
et **en ce que** le boitier d'applicateur (100, 100a) comprend des moyens de contrôle et de commande (22) desdits moyens de déplacement (7, 7a) configurés pour corréler la position du piston (6, 6a) avec la quantité de produit dermatologique restant dans le réservoir (4, 4a).

2. Boitier d'applicateur (100) selon la revendication précédente, **caractérisé en ce que** les moyens de détection de la position du piston (6) sont configurés pour détecter une surconsommation du moteur (11) lorsque l'élément pousse-piston (9) rentre en appui de contact contre le piston (6).

3. Boitier d'applicateur (100a) selon la revendication 1, **caractérisé en ce que** les moyens de détection du piston (6a) comprennent un détecteur de fin de course (30) solidaire de l'extrémité libre (10a) de l'élément pousse-piston (9a) et configuré pour détecter un contact avec le piston (6a).

4. Boitier d'applicateur (100, 100a) selon l'une quelconque des revendications précédentes, **caractérisé en ce que** les moyens de détermination (8) de position du piston (6, 6a) sont configurés pour déterminer la longueur de déplacement de l'élément pousse-piston (9, 9a) entre une position initiale et une position dans laquelle l'élément pousse-piston (9, 9a) est configurée pour être au contact du piston (6, 6a).

5. Boitier d'applicateur (100) selon l'une quelconque des revendications 1 à 3, **caractérisé en ce que** les moyens de détermination (8) de position comprennent un capteur du type codeur optique (12) ménagé sur les moyens de déplacement (7) et configuré pour mesurer la distance parcourue par ledit élément pousse-piston (9).

6. Boitier d'applicateur (100, 100a) selon l'une quelconque des revendications précédentes, **caractérisé en ce que** les moyens de détermination (8) de la position comprennent un aimant et un capteur du type à effet Hall (13) de lecture de l'aimant solidaire de l'élément pousse-piston (9, 9a).

7. Boitier d'applicateur (100, 100a) selon l'une quelconque des revendications précédentes, **caractérisé en ce que** l'élément pousse-piston (9, 9a) est monté flottant sur un ressort (14, 14a).

8. Boitier d'applicateur (100) selon la revendication précédente, **caractérisé en ce que** l'élément pousse-piston (9) est monté autour d'au moins une tige de guidage (15).

9. Boitier d'applicateur (100, 100a) selon l'une quelconque des revendications précédentes, **caractérisé en ce que** les moyens de déplacement (7, 7a) comprennent un élément pousseur (28, 28a) relié à l'élément pousse-piston (9, 9a) et un moteur (11, 11a) comportant un axe de rotation fileté (27, 27a), lequel élément pousseur (28, 28a) comprend une portion taraudée montée sur l'axe fileté (27, 27a) et une fourche radiale anti rotation (40) coopérant avec un élément anti-rotation (42, 15) solidaire du boitier d'applicateur (100, 100a).

10. Applicateur (1, 1a) comprenant un boitier d'applicateur (100, 100a) selon l'une quelconque des revendication 1 à 9 et une capsule (16, 16a) intégrée de manière amovible dans le corps principal (2, 2a) et comprenant une tête d'application de produit (3, 3a) située au niveau de l'extrémité libre du corps principal (2, 2a), et un réservoir de produit dermatologique (4, 4a) relié à la tête d'application (3, 3a) et comprenant, au niveau de l'une de ses extrémités, une sortie (5, 5a) de produit débouchant en vis-à-vis de la tête d'application (3, 3a) et, au niveau de son extrémité opposée, un piston (6, 6a) formant le fond du réservoir (4, 4a), lequel piston (6, 6a) est déplaçable en translation dans le réservoir (4, 4a) selon l'axe longitudinal (X) pour assurer la sortie de produit dermatologique hors dudit réservoir (4, 4a) vers la tête d'application (3, 3a).

11. Procédé de contrôle de la quantité d'un produit dermatologique contenu dans un réservoir muni d'un piston (6, 6a) d'une capsule (16, 16a) pour un applicateur rechargeable de produit dermatologique (1, 1a), lequel applicateur (1, 1a) comprend un corps principal (2, 2a) s'étendant selon un axe longitudinal (X) et des moyens de déplacement (7, 7a) motorisés du piston (6, 6a) du réservoir (4, 4a) ménagés dans le corps principal (2, 2a) et comportant un élément pousse-piston (9, 9a) déplaçable en translation selon l'axe longitudinal (X) et dont l'extrémité libre (10, 10a) est prévue pour venir en appui de contact contre le piston (6, 6a) du réservoir (4, 4a), lequel procédé comprend les étapes successives de :
• Insertion d'une capsule (16, 16a) de produit dermatologique dans le corps principal de l'applicateur, la capsule (16, 16a) comportant une tête d'application (3, 3a) de produit ménagée au niveau d'une extrémité libre du corps principal (2, 2a) et le réservoir de produit (4, 4a) relié à la tête d'application et comprenant, au niveau d'une de ses extrémités, une sortie de produit (5, 5a) et, au niveau de son extrémité opposée, le piston (6, 6a) formant le fond du réservoir (4, 4a), lequel piston (6, 6a) est déplaçable en translation dans le réservoir (4, 4a) selon l'axe longitudinal (X) pour assurer la sortie de produit hors dudit réservoir (4, 4a) ;
• Actionnement, par des moyens de commande de l'applicateur (1, 1a), des moyens de déplacement (7, 7a) en direction du piston (6, 6a) ;
• Détection du contact entre les moyens de déplacement (7, 7a) et le piston (6, 6a), suivi de l'arrêt des moyens de déplacement (7, 7a) ;
• Détermination de la position du piston (6, 6a) et de la quantité de produit cosmétique dans le réservoir (4, 4a) à partir de ladite position du piston (6, 6a), quelle que soit la position dudit piston (6, 6a) dans le réservoir (4, 4a).

12. Procédé selon la revendication précédente, **caractérisé en ce qu'**il comprend une étape de déplacement des moyens de déplacement (7, 7a) vers une position initiale dès que le réservoir (4, 4a) est dégagé du corps principal.

13. Procédé selon la revendication 11 ou 12, **caractérisé en ce que** l'étape de détermination de la position du piston (6, 6a) comprend les sous-étapes de :
• Détermination de la distance parcourue par les moyens de déplacement (7, 7a) entre leur position initiale et leur position de contact avec le piston (6, 6a) ;
• Corrélation par les moyens de contrôle et de commande entre la distance parcourue par les moyens de déplacement (7, 7a) et une base de données enregistrée dans un espace mémoire des moyens de contrôle et de commande (22) pour déterminer la position du piston (6, 6a) et la quantité de produit restant dans le réservoir (4, 4a).

14. Procédé selon l'une quelconque des revendications 11 à 13, **caractérisé en ce que** l'applicateur (1, 1a) comprend des moyens de détection du piston (6, 6a) et de détermination de sa position dans le réservoir (4, 4a), et **en ce que** l'actionnement des moyens de déplacement (7, 7a) en direction du piston (6, 6a) comprennent les sous-étapes successives de :
• Détermination préliminaire de la position du piston (6, 6a) dans le réservoir (4, 4a) ;
• Actionnement et déplacement des moyens de déplacement (7, 7a) selon une première vitesse déterminée ;
• Détection des moyens de déplacement (7, 7a) à une distance du piston (6, 6a) inférieure à une valeur déterminée, et
• Diminution de la vitesse de progression des moyens de déplacement (7, 7a) jusqu'à une seconde vitesse déterminée.

## Patentansprüche

1. Nachfüllbares Applikatorgehäuse (100, 100a) für ein dermatologisches Produkt, das sich entlang einer Längsachse (X) erstreckt und dazu ausgelegt ist, eine Kapsel (16, 16a) abnehmbar aufzunehmen, die einen Produktapplikationskopf (3, 3a) umfasst, der sich an einem freien Ende des Gehäuses befindet (100, 100a) angeordnet ist, sowie einen mit dem Applikationskopf (3, 3a) verbundenen Behälter (4, 4a) für ein dermatologisches Produkt, der an einem seiner Enden einen Produktauslass (5, 5a) aufweist, der gegenüber dem Applikationskopf (3, 3a) mündet, und an seinem gegenüberliegenden Ende einen Kolben (6, 6a), der den Boden des Behälters (4, 4a) bildet, wobei dieser Kolben (6, 6a) im Behälter (4, 4a) entlang der Längsachse (X) verschiebbar ist, um den Austritt des Produkts aus dem Behälter (4, 4a) zum Applikationskopf (3, 3a) zu gewährleisten, wobei das Gehäuse (100, 100a) umfasst:
• einen sich entlang der Längsachse (X) erstreckenden Hauptkörper (2, 2a) sowie
• motorisierte Verschiebungsmittel (7, 7a) für den Kolben (6, 6a), die im Hauptkörper (2, 2a) angeordnet sind und ein Kolben-Schubelement (9, 9a), das entlang der Längsachse (X) verschiebbar ist und dessen freies Ende (10, 10a) so ausgelegt ist, dass es in Kontakt mit dem Kolben (6, 6a) des Behälters (4, 4a) steht,
**dadurch gekennzeichnet, dass**
die Verschiebungsmittel (7, 7a) Mittel zur Erfassung des Kolbens (6, 6a) der Kapsel (16, 16a) und zur Bestimmung (8) seiner Position im Behälter (4, 4a) umfassen, wenn das Kolbenschubelement (9, 9a) mit dem Kolben (6, 6a) in Kontakt steht, unabhängig von der Position des Kolbens (6, 6a) im Behälter (4, 4a),
und dass das Applikatorgehäuse (100, 100a) Mittel zur Überwachung und Steuerung (22) der genannten Verschiebungsmittel (7, 7a) umfasst, die so ausgelegt sind, dass sie die Position des Kolbens (6, 6a) mit der im Behälter (4, 4a) verbleibenden Menge an dermatologischem Produkt in Beziehung setzen.

2. Applikatorgehäuse (100) nach dem vorstehenden Anspruch, **dadurch gekennzeichnet, dass** die Mittel zur Erfassung der Position des Kolbens (6) so ausgelegt sind, dass sie einen übermäßigen Verbrauch des Motors (11) erfassen, wenn das Kolbendrückelement (9) in Kontakt mit dem Kolben (6) kommt.

3. Applikatorgehäuse (100a) nach Anspruch 1, **dadurch gekennzeichnet, dass** die Mittel zur Erfassung des Kolbens (6a) einen Endschalter (30) umfassen, der fest mit dem freien Ende (10a) des Kolbenschieberelements (9a) verbunden und so ausgelegt ist, dass er einen Kontakt mit dem Kolben (6a) zu erfassen.

4. Applikatorgehäuse (100, 100a) nach einem der vorstehenden Ansprüche, **dadurch gekennzeichnet, dass** die Mittel (8) zur Bestimmung der Position des Kolbens (6, 6a) so ausgelegt sind, dass sie die Verfahrlänge des Kolbenschieberelements (9, 9a) zwischen einer Ausgangsposition und einer Position zu ermitteln, in der das Kolbenschubelement (9, 9a) so ausgelegt ist, dass es mit dem Kolben (6, 6a) in Kontakt steht.

5. Applikatorgehäuse (100) nach einem der Ansprüche 1 bis 3, **dadurch gekennzeichnet, dass** die Positionsermittlungsmittel (8) einen Sensor vom Typ eines optischen Encoders (12) umfassen, der an den Verschiebungsmitteln (7) angeordnet und so ausgelegt ist, dass er die von dem Kolben-Schiebeelement (9) zurückgelegte Strecke misst.

6. Applikatorgehäuse (100, 100a) gemäß einem der vorstehenden Ansprüche, **dadurch gekennzeichnet, dass** die Positionsermittlungsmittel (8) einen Magneten und einen Hall-Sensor (13) zum Abtasten des Magneten umfassen, der fest mit dem Kolbenstößel (9, 9a) verbunden ist.

7. Applikatorgehäuse (100, 100a) gemäß einem der vorstehenden Ansprüche, **dadurch gekennzeichnet, dass** das Kolbenschubelement (9, 9a) schwimmend auf einer Feder (14, 14a) gelagert ist.

8. Applikatorgehäuse (100) nach dem vorstehenden Anspruch, **dadurch gekennzeichnet, dass** das Kolbenschubelement (9) um mindestens eine Führungsstange (15) herum angebracht ist.

9. Applikatorgehäuse (100, 100a) nach einem der vorstehenden Ansprüche, **dadurch gekennzeichnet, dass** die Verstellvorrichtungen (7, 7a) ein mit dem Kolbenschieber (9, 9a) verbundenes Schiebeelement (28, 28a) und einen Motor (11, 11a) mit einer mit Gewinde versehenen Drehachse (27, 27a) aufweist, wobei das Schubelement (28, 28a) einen auf der Gewindeachse (27, 27a) montierten Gewindeabschnitt und eine radiale Verdrehsicherung (40) umfasst, die mit einem Verdrehsicherungselement (42, 15) zusammenwirkt, das fest mit dem Applikatorgehäuse (100, 100a) verbunden ist.

10. Applikator (1, 1a) mit einem Applikatorgehäuse (100, 100a) gemäß einem der Ansprüche 1 bis 9 und eine Kapsel (16, 16a), die abnehmbar in den Hauptkörper (2, 2a) integriert ist und einen Produktapplikationskopf (3, 3a) umfasst, der sich am freien Ende des Hauptkörpers (2, 2a) befindet, sowie einen Behälter für ein dermatologisches Produkt (4, 4a), der mit dem Auftragskopf (3, 3a) verbunden ist und an einem seiner Enden einen Produktauslass (5, 5a) aufweist, der gegenüber dem Auftragskopf (3, 3a) mündet, und an seinem gegenüberliegenden Ende einen Kolben (6, 6a), der den Boden des Behälters (4, 4a) bildet, wobei dieser Kolben (6, 6a) im Behälter (4, 4a) entlang der Längsachse (X) verschiebbar ist, um den Austritt des dermatologischen Produkts aus dem Behälter (4, 4a) zum Auftragskopf (3, 3a) zu gewährleisten.

11. Verfahren zur Steuerung der Menge eines in einem mit einem Kolben (6, 6a) versehenen Behälter einer Kapsel (16, 16a) enthaltenen dermatologischen Produkts für einen nachfüllbaren Applikator (1, 1a) für dermatologische Produkte, wobei der Applikator (1, 1a) einen Hauptkörper (2, 2a), der sich entlang einer Längsachse (X) erstreckt, sowie motorisierte Verschiebungsmittel (7, 7a) für den Kolben (6, 6a) des Behälters (4, 4a) umfasst, die im Hauptkörper (2, 2a) angeordnet sind und ein KolbenSchubelement (9, 9a) aufweist, das entlang der Längsachse (X) verschiebbar ist und dessen freies Ende (10, 10a) dazu vorgesehen ist, in Kontakt mit dem Kolben (6, 6a) des Behälters (4, 4a) zu kommen, wobei das Verfahren die folgenden aufeinanderfolgenden Schritte umfasst:
• Einführen einer Kapsel (16, 16a) mit einem dermatologischen Produkt in den Hauptkörper des Applikators, wobei die Kapsel (16, 16a) einen Applikationskopf (3, 3a) aufweist, der an einem freien Ende des Hauptkörpers (2, 2a) angeordnet ist, und wobei der Produktbehälter (4, 4a) mit dem Applikationskopf verbunden ist und an einem seiner Enden einen Produktauslass (5, 5a) sowie an seinem gegenüberliegenden Ende den Kolben (6, 6a), der den Boden des Vorratsbehälters (4, 4a) bildet, wobei dieser Kolben (6, 6a) im Vorratsbehälter (4, 4a) entlang der Längsachse (X) verschiebbar ist, um den Austritt des Produkts aus dem genannten Vorratsbehälter (4, 4a) zu gewährleisten;
• Betätigung der Verschiebungsmittel (7, 7a) in Richtung des Kolbens (6, 6a) durch Steuermittel des Applikators (1, 1a);
• Erfassung des Kontakts zwischen den Verschiebungsmitteln (7, 7a) und dem Kolben (6, 6a), gefolgt vom Anhalten der Verschiebungsmittel (7, 7a);
• Ermittlung der Position des Kolbens (6, 6a) und der Menge an Kosmetikprodukt im Behälter (4, 4a) anhand der genannten Position des Kolbens (6, 6a), unabhängig von der Position des Kolbens (6, 6a) im Behälter (4, 4a).

12. Verfahren nach dem vorstehenden Anspruch, **dadurch gekennzeichnet, dass** es einen Schritt umfasst, bei dem die Verstellvorrichtungen (7, 7a) in eine Ausgangsposition bewegt werden, sobald der Behälter (4, 4a) vom Hauptkörper gelöst ist.

13. Verfahren nach Anspruch 11 oder 12, **dadurch gekennzeichnet, dass** der Schritt zur Bestimmung der Position des Kolbens (6, 6a) die folgenden Teilschritte umfasst:
• Ermittlung der von den Bewegungsmitteln (7, 7a) zwischen ihrer Ausgangsposition und ihrer Kontaktposition mit dem Kolben (6, 6a) zurückgelegten Strecke;
• Abgleich durch die Steuer- und Regelmittel zwischen der von den Verschiebungsmitteln (7, 7a) zurückgelegten Strecke und einer in einem Speicherbereich der Steuer- und Regelmittel (22) gespeicherten Datenbank, um die Position des Kolbens (6, 6a) und die im Behälter (4, 4a) verbleibende Produktmenge zu bestimmen.

14. Verfahren nach einem der Ansprüche 11 bis 13, **dadurch gekennzeichnet, dass** der Applikator (1, 1a) Mittel zur Erfassung des Kolbens (6, 6a) und zur Bestimmung seiner Position im Behälter (4, 4a) umfasst, und dadurch, dass die Betätigung der Verschiebungsmittel (7, 7a) in Richtung des Kolbens (6, 6a) die folgenden aufeinanderfolgenden Teilschritte umfasst:
• Vorläufige Bestimmung der Position des Kolbens (6, 6a) im Behälter (4, 4a);
• Betätigung und Bewegung der Bewegungsmittel (7, 7a) mit einer ersten festgelegten Geschwindigkeit;
• Erfassung der Verschiebungsmittel (7, 7a) in einem Abstand zum Kolben (6, 6a), der kleiner ist als ein vorbestimmter Wert, und
• Verringerung der Vorschubgeschwindigkeit der Verschiebeeinrichtungen (7, 7a) auf eine zweite festgelegte Geschwindigkeit.

## Claims

1. A refillable applicator casing (100, 100a) for a dermatological product, extending along a longitudinal axis (X), capable of removably accommodating a cartridge (16, 16a) comprising a product application head (3, 3a) located at a free end of the casing (100, 100a), and a reservoir (4, 4a) for the dermatological product connected to the applicator head (3, 3a) and comprising, at one of its ends, a product outlet (5, 5a) opening facing the applicator head (3, 3a) and, at its opposite end, a piston (6, 6a) forming the base of the reservoir (4, 4a), which piston (6, 6a) is capable of translational movement within the reservoir (4, 4a) along the longitudinal axis (X) to ensure the discharge of product from the reservoir (4, 4a) towards the application head (3, 3a), which housing (100, 100a) comprises:
• a main body (2, 2a) extending along the longitudinal axis (X), and
• motorized piston displacement means (7, 7a) for the piston (6, 6a) provided within the main body (2, 2a) and comprising a piston-pushing element (9, 9a) capable of translational movement along the longitudinal axis (X), the free end (10, 10a) of which is designed to bear in contact against the piston (6, 6a) of the reservoir (4, 4a),
**characterized in that**
the displacement means (7, 7a) comprise means for detecting the piston (6, 6a) of the capsule (16, 16a) and for determining (8) its position within the reservoir (4, 4a) when the piston-pushing element (9, 9a) is in contact with the piston (6, 6a), regardless of the position of said piston (6, 6a) within the reservoir (4, 4a),
and **in that** the applicator housing (100, 100a) comprises means (22) for monitoring and controlling said displacement means (7, 7a), configured to correlate the position of the piston (6, 6a) with the quantity of dermatological product remaining in the reservoir (4, 4a).

2. Applicator casing (100) according to the preceding claim, **characterized in that** the means for detecting the position of the piston (6) are configured to detect excessive power consumption by the motor (11) when the piston-pushing element (9) comes into contact with the piston (6).

3. An applicator casing (100a) according to claim 1, **characterized in that** the means for detecting the position of the piston (6a) comprise a limit switch (30) integral with the free end (10a) of the piston-pushing element (9a) and configured to detect contact with the piston (6a).

4. An applicator casing (100, 100a) according to any one of the preceding claims, **characterized in that** the means (8) for determining the position of the piston (6, 6a) are configured to determine the length of travel of the piston-pushing element (9, 9a) between an initial position and a position in which the piston-pushing element (9, 9a) is configured to be in contact with the piston (6, 6a).

5. An applicator casing (100) according to any one of claims 1 to 3, **characterized in that** the position-determining means (8) comprise an optical encoder-type sensor (12) provided on the displacement means (7) and configured to measure the distance travelled by said piston-pushing element (9).

6. An applicator casing (100, 100a) according to any one of the preceding claims, **characterized in that** the position-detection means (8) comprise a magnet and a Hall-effect sensor (13) for detecting the magnet, which is integral with the piston-pushing element (9, 9a).

7. An applicator casing (100, 100a) according to any one of the preceding claims, **characterized in that** the piston-pushing element (9, 9a) is mounted in a floating manner on a spring (14, 14a).

8. An applicator casing (100) according to the preceding claim, **characterized in that** the piston-pushing element (9) is mounted around at least one guide rod (15).

9. An applicator casing (100, 100a) according to any one of the preceding claims, **characterized in that** the displacement means (7, 7a) comprise a pusher element (28, 28a) connected to the piston-pushing element (9, 9a) and a motor (11, 11a) comprising a threaded rotary shaft (27, 27a), which pusher element (28, 28a) comprises a threaded portion mounted on the threaded shaft (27, 27a) and a radial anti-rotation fork (40) cooperating with an anti-rotation element (42, 15) integral with the applicator housing (100, 100a).

10. Applicator (1, 1a) comprising an applicator housing (100, 100a) according to any one of claims 1 to 9 and a capsule (16, 16a) removably integrated into the main body (2, 2a) and comprising a product application head (3, 3a) located at the free end of the main body (2, 2a), and a reservoir for a dermatological product (4, 4a) connected to the applicator head (3, 3a) and comprising, at one of its ends, a product outlet (5, 5a) opening facing the applicator head (3, 3a) and, at its opposite end, a piston (6, 6a) forming the base of the reservoir (4, 4a), which piston (6, 6a) is capable of translational movement within the reservoir (4, 4a) along the longitudinal axis (X) to ensure the discharge of the dermatological product from said reservoir (4, 4a) towards the application head (3, 3a).

11. A method for controlling the quantity of a dermatological product contained in a reservoir fitted with a piston (6, 6a) of a cartridge (16, 16a) for a refillable dermatological product applicator (1, 1a), which applicator (1, 1a) comprises a main body (2, 2a) extending along a longitudinal axis (X) and motorized means (7, 7a) for moving the piston (6, 6a) of the reservoir (4, 4a), which are provided within the main body (2, 2a) and comprise a piston-pushing element (9, 9a) capable of translational movement along the longitudinal axis (X), the free end (10, 10a) of which is designed to bear in contact against the piston (6, 6a) of the reservoir (4, 4a), which method comprises the successive steps of:
• Inserting a capsule (16, 16a) of dermatological product into the main body of the applicator, the capsule (16, 16a) comprising a product application head (3, 3a) formed at a free end of the main body (2, 2a), and the product reservoir (4, 4a) connected to the application head and comprising, at one of its ends, a product outlet (5, 5a) and, at its opposite end, the piston (6, 6a) forming the base of the reservoir (4, 4a), which piston (6, 6a) is capable of translational movement within the reservoir (4, 4a) along the longitudinal axis (X) to ensure the discharge of product from said reservoir (4, 4a);
• Actuation, by control means of the applicator (1, 1a), of displacement means (7, 7a) towards the piston (6, 6a);
• Detection of contact between the displacement means (7, 7a) and the piston (6, 6a), followed by the stopping of the displacement means (7, 7a);
• Determining the position of the piston (6, 6a) and the quantity of cosmetic product in the reservoir (4, 4a) based on said position of the piston (6, 6a), regardless of the position of said piston (6, 6a) within the reservoir (4, 4a).

12. A method according to the preceding claim, **characterized in that** it comprises a step of moving the displacement means (7, 7a) to an initial position as soon as the reservoir (4, 4a) is disengaged from the main body.

13. A method according to claim 11 or 12, **characterized in that** the step of determining the position of the piston (6, 6a) comprises the sub-steps of:
• Determining the distance travelled by the displacement means (7, 7a) between their initial position and their position of contact with the piston (6, 6a);
• Correlating, by the control and command means, the distance travelled by the displacement means (7, 7a) with a database stored in a memory space of the control and command means (22) to determine the position of the piston (6, 6a) and the quantity of product remaining in the reservoir (4, 4a).

14. A method according to any one of claims 11 to 13, **characterized in that** the applicator (1, 1a) comprises means for detecting the piston (6, 6a), and for determining its position within the reservoir (4, 4a), and **in that** the actuation of the displacement means (7, 7a) towards the piston (6, 6a) comprises the successive sub-steps of:
• Preliminary determination of the position of the piston (6, 6a) within the reservoir (4, 4a);
• Actuating and moving the displacement means (7, 7a) at a first predetermined speed;
• Detecting that the displacement means (7, 7a) are at a distance from the piston (6, 6a) that is less than a predetermined value, and
• Reducing the speed of the displacement means (7, 7a) to a second predetermined speed.
